# EUROPEAN PATENT SPECIFICATION

(11) **EP 0 507 292 B1**
(45) Date of publication and mention of the grant of the patent: **02.07.1997**
(21) Application number: 92105656.0
(22) Date of filing: 02.04.1992
(51) Int. Cl.: A61F 2/16, A61F 2/14

(54) **Device for inhibiting aftercataract**
Vorrichtung zum Verhindern von Sekundärkatarakt
Dispositif inhibiteur de la cataracte secondaire

(30) Priority: 04.04.1991 JP 71894/91; 06.11.1991 JP 289940/91; 22.01.1992 JP 1826/92
(43) Date of publication of application: 07.10.1992
(73) Proprietor: Menicon Co., Ltd., Nagoya-shi Aichi-ken (JP)
(72) Inventor: Hara, Tsutomu, Utsunomiya-shi, Tochigi-ken (JP); Hara, Takako, Utsunomiya-shi, Tochigi-ken (JP); Yamada, Yoshiharu, c/o Noritake Fac. Menicon Co., Nagoya-shi, Aichi-ken (JP); Mizumoto, Yuriko, c/o Noritake Fac. Menicon Co., Nagoya-shi, Aichi-ken (JP); Nagai, Hidenobu, c/o Noritake Fac. Menicon Co., Nagoya-shi, Aichi-ken (JP)
(74) Representative: Türk, Gille, Hrabal, Leifert

(56) References cited:
- WO-A-89/00032
- WO-A-89/07426
- FR-A- 2 530 457
- US-A- 4 764 169
- US-A- 4 946 469

## Description

The present invention relates to a device for inhibiting aftercataract (hereinafter referred to as an inhibiting device) and more particularly to an inhibiting device for keeping the shape of a capsular bag substantially circular after a cataract extraction and for inhibiting such as invasion metamorphosed epithelial cells into a posterior capsular bag, and further to an inhibiting device wherein an intraocular lens can be retained in good state by forming a groove in the inner periphery thereof.

As an operation method of cataract, the method, wherein an anterior capsular bag is provided with an opening and the crystalline lens is removed through the opening, has hitherto been employed. The refraction of the eye is rectified by inserting an intraocular lens into the capsular bag instead of the removed crystalline lens, by wearing a contact lens on a cornea, or by wearing a pair of spectacles.

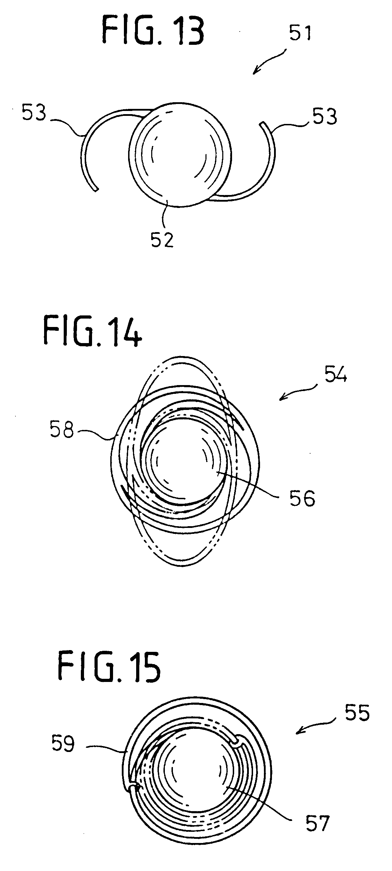

When the intraocular lens is used, such an intraocular lens 51 as shown in Fig. 13 has hitherto been used. The intraocular lens is composed of a lens 52 and two support members 53 having a wire like shape. The support member 53, wherein one end is fixed to the lens 52 and another end (outer side) is bended in such a manner as to extend along the equator of the capsular bag and contact with the equator, keeps the equator of the capsular bag circular. However, it is difficult that the above mentioned support member 53 keeps the equator of the capsular bag circular due to the shape thereof, and there is such a danger that the support member adds an uneven force by partially contacting with the equator. Therefore, after inserting the intraocular lens into the capsular bag, the intraocular lens in the capsular bag becomes unstable after a long interval. Then, deviation or falling down sometimes happens.

The intraocular lenses 54, 55 shown in Figs. 14 and 15 are proposed (with reference to JP-A-285258/1989 and JP-A-503525/1990). Each of the above lenses is composed of a lens portion 56, 57 and a support member 58, 59 which encloses the outer periphery of the lens portion having a circular loop-like shape. The equator of the capsular bag can be kept to be circular in more preferable state compared with the intraocular lens of Fig. 13. It is difficult to insert the circular support member into the capsular bag through the incised opening as the original shape thereof. Therefore, the intraocular lens is inserted into the capsular bag by deflecting the support member to have an oval shape (referring to Fig. 14) or shrinking the support member in such a manner that circular shape thereof is kept (referring to Fig. 15).

US-A-4 764 169 discloses an intraocular lens for placement in a capsular bag through a small incision in the eye, including a small hard inner lens optic and a soft pliable skirt surrounding the lens optic. The optical member located in the central part of the intraocular lens and the supporting member are constructed in a manner as to be integrated with each other. The yag space lens is self-centering and does not require any additional supporting structures.

US-A-4 946 469 discloses an intraocular lens having support means allowing the lens to be held within the natural lens capsule in position on axis within the eye. The known intraocular lens may be inserted into the eye through an opening of dimensions much smaller than that of the lens itself and does not cause irritation or other harm to the eye.

It is further known (FR-2 530 457 A) to provide an intraocular lens with a ring-like circular support having a circular cross-section, the intraocular lens being connected to the support by means of a foil or by means of threads. The support is adapted to be loosely inserted into the equator of a capsular bag of a human eye thus that the circular support does not actively touch the equator of the capsular bag.

The capsular bag after a cataract extraction sometimes happens to produce opacity by proliferation and denaturation of epithelial cells or metaplasia of partial epithelial cells residual in the equator of the capsular bag if the capsular bag is left as it is. This phenomenon is generally called an aftercataract. A secondary operation, dilaceratio, need to be performed. As mentioned hereinbefore, when the intraocular lens is inserted into the capsular bag and the intraocular lens is fixed therein after the cataract extraction, there is such an intraocular lens as to inhibit the aftercataract to a certain extent by keeping the shape of the capsular bag and sealing the residual epithelial cell in the equator by virtue of the shape of the support member of the intraocular lens. However, the support member is not coming into contact with the whole part of the equator. Therefore, aftercatarct is not completely inhibited.

Further, when the capsular bag which is left as it is for a long time not inserting the intraocular lens, that is, the eyesight is rectified in such a manner that the contact lens is worn or the pair of spectacles is worn, the capsular bag, wherein cataract extraction is performed, aftercataract and the like is often produced. When the capsular bag is removed, such a complication that the vitreous body located in the back side of the eye goes forward is apt to come out. Accordingly, the capsular bag is not removed even if the intraocular lens is not inserted into the capsular bag.

The object of the present invention is to resolve the problems mentioned hereinbefore and to provide an inhibiting device wherein aftercataract is inhibited from coming out and the circular shape of the equator can be kept stably.

This object is solved by a device for inhibiting aftercataract comprising the features of Claim 1.

This device for inhibiting aftercataract is made of a material having a resilient property and has a substantially ring-like shape so as to be internally touchable to the equator of a capsular bag along the whole outer periphery thereof.

Further, it is preferable that the device is provided with a groove for engaging with a support member of an intraocular lens extending in the circumferential direction at inner periphery of the device in order to retain the intraocular lens.

In this case, it is preferable that the groove is formed in the whole inner periphery of the device.

Further, it is preferable that the groove comprises a front wall portion and a back wall portion forming protrusions circumferentially in the inner periphery of the device in such a manner that the back wall portion is extending more toward the center of the device than the front wall portion, and said back wall portion being bevelled in such a manner that the width in the axial direction (or the widthwise direction) of the device is widened as the back wall portion is away from the center of the device.

The meanings of "substantially has a circular shape" mentioned in Claims is a concept including not only a complete round but also a polygonal shape so that the outer periphery of the device can be internally touched to the whole of the equator of the capsular bag. The meanings of "a front wall portion" is one of side walls located in the front side of the divice when the device is worn in the eye and the meanings of "a back wall portion" is the other side wall located in the back side of the capsular bag of the eye.

The inhibiting device of the present invention is easily deformed in accordance with the desired shape by pinching the device slightly by means of a pincette and the like. Therefore, particular technique for inserting is not required and the device is easily inserted into the capsular bag through even a small incised opening by means of Continuous Circular Capsulorhexis (CCC) method. After inserting the device into the capsular bag, the device returns to the circular shape, which is an original shape, by means of the resilient property of the device. Accordingly, the outer periphery of the device is internally touched to the whole of the equator and the epithelial cells which are residual within the portion between the equator and the outer periphery of the device, so that proliferation of the residual epithelial cells can be inhibited in good condition. On the other hand, the equator of the capsular bag can be kept to be almost circular. Therefore, the capsular bag is given to a tensity so that the capsular bag is effectively inhibited from shrinking. Thus, formation of aftercataract can be inhibited by the effect of the device.

Further, the shape of the incised opening is kept and the intraocular lens can be easily inserted into the capsular bag by the tension of the capsular bag. In this case, when the inhibiting device having a groove in the inner periphery thereof is used, the support portion of the intraocular lens inserted into the capsular bag is engaged with the groove formed in the inner periphery of the inhibiting device and fixed thereto. As a result, the intraocular lens is inhibiting from falling down or deviation.
Fig. 1 is a partially cutaway perspective view showing an embodiment of an inhibiting device of the present invention;
Fig. 2 is a partially schematic sectional view taken along lines II-II in Fig. 1;
Fig. 3 is a partially schematic sectional view showing another embodiment of the inhibiting device of the present invention;
Fig. 4 is a partially schematic sectional view showing another embodiment of the inhibiting device of the present invention;
Fig. 5 is an explaining view showing a use of the inhibiting device of Fig. 1;
Fig. 6 is an explaining view showing a use of the inhibiting device of Fig. 1;
Fig. 7 is an explaining view showing a use of the inhibiting device of Fig. 1;
Fig. 8 is an explaining view showing a use of the inhibiting device of Fig. 1;
Fig. 9 is a partially schematic sectional view showing another embodiment of the imhibiting device of the present invention;
Fig. 10 is an explaining view showing a use of the inhibiting device of Fig. 9;
Fig. 11 is a perspective view showing another embodiments of the inhibiting device of the present invention;
Fig. 12 is an explaining view showing a use of the inhibiting device of Fig. 11;
Fig. 13 is a plan view showing an example of a conventional intraocular lens;
Fig. 14 is a plan view showing another example of a conventional intraocular lens; and
Fig. 15 is a plan view showing another example of a conventional intraocular lens.

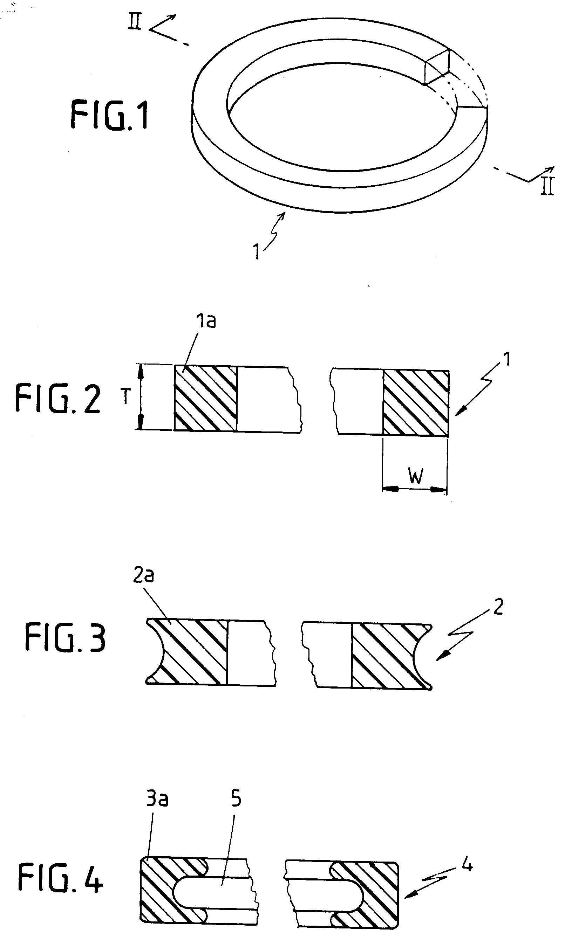

In Fig. 1, a ring-like inhibiting device 1 is shown, wherein the outer diameter is about 10 mm, the inner diameter is about 8 mm and each of width (W in Fig. 2) and thickness (T in Fig. 2) is, respectively, about 1 mm. Those sizes are values which are determined by a normal size of the capsular bag. Accordingly, the outer diameter and the thickness are not limited to the above size. For instance, with respect to the outer diameter, a range of 8 to 12 mm is general and with respect to the thickness, a range of 0.4 to 2.0 mm is general. Further, there is such a case as to be beside the above range in accordance with the material which forms the inhibiting device. The shape in section of a base wire portion of the inhibiting device is substantially square, wherein the length of each side is about 1 mm, as shown in Fig. 2. In the present invention, any shape in section of the outer periphery of the inhibiting device 1 can be employed, wherein the residual epithelial cells can be sealed in the equator in good state. The shape in section is not limited to a square shape; such a shape as shown in Fig. 3 can be employed.

In the inhibiting device 1 having a square shape in section as shown in Fig. 2, corner portions 1a forming protrusions along the whole outer periphery which are coming into substantially line contact with the capsular bag. Accordingly, the residual epithelial cells can be sealed in the equator of the capsular bag in good state and the capsular bag can be prevented from producing an aftercataract caused by a proliferation or denaturation of the residual epithelial cells or by a mataplasia of a part of the residual epithelial cells. Then, the aftercataract which becomes a problem after removing the crystalline lens can be effectively inhibited. The inhibiting device 2 shown in Fig. 3 has also corner portions 2a wherein protrusions are formed along the whole outer periphery. Therefore, the protrusions of the corner portions 2a cause the same effect that the protrusions 1a cause. The inhibiting device 2 has a shape wherein the outer periphery has a recess which is grooved centripetally between the corner portions 2a. Therefore, when into the eye the inhibiting device 2 is inserted as mentioned hereinafter, the inhibiting device can be easily bended by engaging a pincette and the like with the recess.

On the other hand, in Fig. 4 an inhibiting device 4, wherein a groove 5 for retaining a support portion of an intraocular lens is formed in the whole inner periphery, is shown. The width W of the groove 5 is about 0.5 mm and the depth T of the groove 5 is about 0.5 mm. The size of the groove 5 is also determined by the size of the support portion. Accordingly, in the inhibiting device of the present invention, the size of the groove is not limited to the above value. Further, in the present invention, the groove is not necessarily limited in such a manner that the groove is formed in the whole of the inner periphery, such a shape that the groove is partially or intermittently formed can be employed in accordance with the shape of the support portion of the intraocular lens which is engaged with the inhibiting device. However, the groove 5 which is formed in the whole inner periphery is preferably employed in order to cope with all kinds of intraocular lenses and all kinds of methods for inserting the intraocular lens.

According to the conventional intraocular lenses 54, 55 (referring to Figs. 14 and 15), even if the intraocular lens is made to be such a means as to be easily inserted into the capsular bag, when the intraocular lens 54 shown in Fig. 14 is inserted into the capsular bag, the support portion of the intraocular lens is permitted to become an oval-like shape (shown with two-dot chain line in Fig. 14) so that the major axis of the oval is longer than the inner diameter (about 10 mm in a normal case) of the capsular bag. Accordingly, the intraocular lens 54 is difficult to be inserted into the capsular bag so that the intraocular lens has no practical use. On the other hand, with respect to the intraocular lens 55 shown in Fig. 15, such a particular technique of inserting the intraocular lens into the capsular bag has been required that the intraocular lens is shrunk (i.e. the diameter of the intraocular lens is shortened) by winding the support portion 59 along the periphery of the lens portion 57 and the above state of the intraocular lens is kept. Further, the incised opening for inserting the intraocular lens into the capsular bag tends to be small for fear that the eye is damaged. Therefore, it is still more difficult to be inserted.

The inhibiting device of the embodiment of the present invention can resolve the above problems. That is to say, a stably fixed state is ensured by engaging the support portion of the intraocular lens with the groove 5 formed in the inner periphery of the inhibiting device 4 as mentioned hereinbefore.

The shape in section of the inhibiting device is not limited to the circular shape in the present invention. The inhibiting device wherein the shape in section is square as shown in Fig. 4 can be employed.

If the equator is closely contacted with the inhibiting device so that there is not any space to produce the residual epithelial cells in the equator of the capsular bag, the aftercataract can be inhibited.

In the inhibiting device 4 wherein the shape in section is square as shown in Fig. 4, the corner portions 3a located at the outer periphery are coming into substantially line contact with the capsular bag. Therefore, residual epithelial cells can be sealed in the equator of the capsular bag as mentioned hereinbefore, and the effect, wherein aftercataract coming to a postoperative problem is inhibited, is superior.

As material of the inhibiting devices 1, 2 and 4, and the inhibiting devices 11 and 12 of the embodiment mentioned hereinafter, a material which has a resilient property in order to insert the inhibiting device into the capsular bag and stably fix therein, is selected. In the embodiment, silicone rubber is employed as the material. If the material is suitable for ophtalmiatrics (for instance, if the material has a suitability in vivo, a stability of the shape and the like), the material is not limited in the present invention, for instance high polymers having water absorption property comprising hydroxyethylmethacrylate, N-vinylpyrrolidone, vinyl alcohol and the like, a biopolymer comprising collagen and the like, and an elastomer silicone rubber, acrylic rubber and the like and a mixture or copolymer of these materials can be employed.

Further, such a synthetic resin having an inferior clarification as to be normally used as a medical material such as polyethylene, polypropylene vinyl chloride, polyvinylidenefluoride and the like can be employed, and even a hard synthetic resin such as polymethylmethacrylate can be employed as the material of the inhibiting device if the resilient property is given by suitably selecting the shape or thickness.

The inhibiting devices 1, 2 and 4 of the above-mentioned enbodiment and the inhibiting devices 11 and 12 of the embodiment mentioned hereinafter, which are constructed by a single material in consideration of an easiness of manufacturing in such a manner as to be integrated, can be easily manufactured by means of molding.

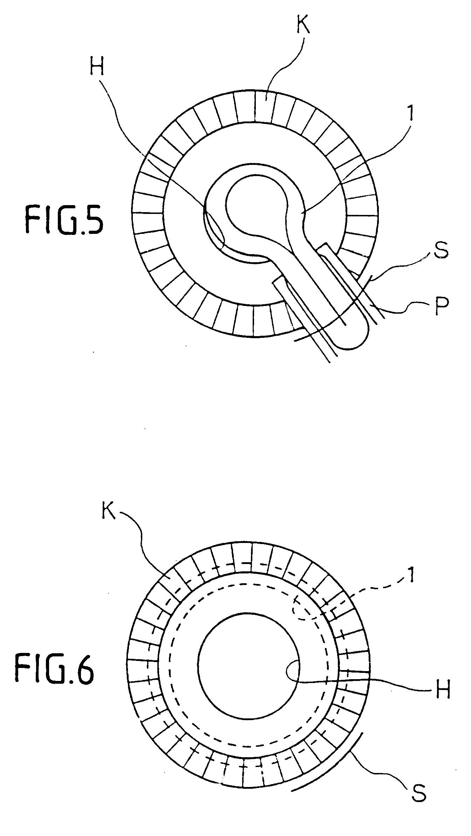

Next, it will be explained with reference to Figs. 5 to 8 how to use the inhibiting device constructed as mentioned hereinbefore. In Figs. 5 to 8, character K denotes a mydriatic iris, character H denotes an incised opening located in the anterior capsular bag, character S denotes an incised opening located in a sclera, character P denotes a pincette.

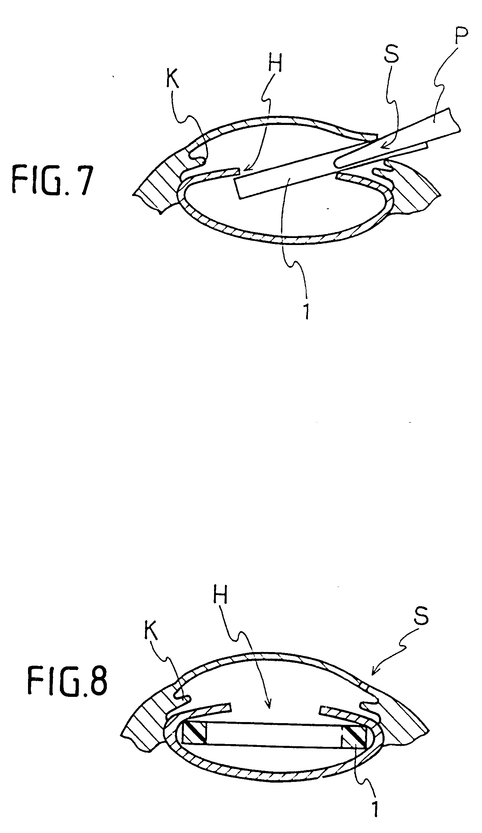

Firstly, a cataractous crystalline lens is removed through the incised opening H and the incised opening S and viscomaterial is fully injected into the capsular bag. Then, the inhibiting device 1 is pinched by the pincette P so that the shape thereof becomes almost oval. Next, the deformed inhibiting device is inserted into the capsular bag through the incised openings S and H (referring to Figs. 5 and 7). In order to insert the inhibiting device 1 into the capsular bag, a tool other than the pincette such as a commercial injector for soft IOL (intraocular lens) can be employed. When the inhibiting device is inserted into the capsular bag, the inhibiting device 1 restores the original circular ring-like shape by means of the resilient property. Accordingly, the inhibiting device is internally touched to the whole of the equator of the capsular bag (referring to Fig. 6 and 8). Thereby, the residual epithelial cells in the equator are sealed therein. Further the capsular bag is given a tension. Therefore, a shrinkage of the capsular bag is effectively inhibited and aftercataract can be inhibited.

Further, the equator is kept to be circular and the incised openings S and H maintain the shape (referring to Figs. 6 and 8). Therefore, the intraocular lens, which is generally used, can be easily inserted into the capsular bag through the incised openings S and H. In this case, if the inhibiting device 4 having a groove is employed, it does not happen that the intraolular lens deviates or falls down since the support portion of the intraocular lens is engaged with the groove of the inhibiting device so that intraocular lens is stably fixed.

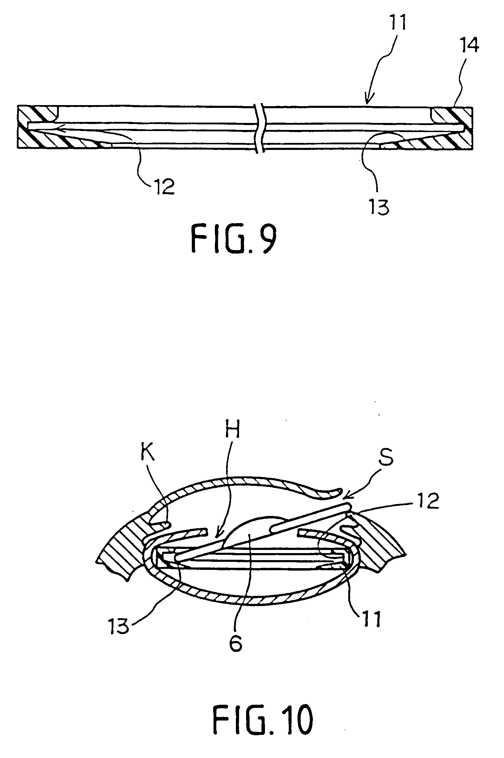

In Fig. 9, another embodiment of the inhibiting device which has a groove in the inner periphery is shown.

The inhibiting device 11 is an example of variation of the inhibiting device 4 having a square shape in section shown in Fig. 4 which has such a construction that a groove 12 comprising a front wall 14 and a back wall 13 is formed in the whole inner periphery of the inhibiting device, the back wall 13 wherein a protrusion which is circumferentially formed in the inner periphery of the inhibiting device extends in a radial direction in such a manner that the back wall 13 is directed to the center of the inhibiting device being still more inner than the front wall 14, and the back wall 13 being bevelled in such a manner that the width of the groove 12 is widened as the back wall 13 is away from the center.

The back wall can be constructed in such a manner that the back wall has a circular shape which is almost concentric to the outline (circular shape) of the inhibiting device when the inhibiting device is viewed from upper side. Such a construction can be also employed that the portion, wherein the support portion of the intraocular lens is contained, is centripetally protruded. In short, such a shape can be employed that an optical portion of the intraocular lens to be inserted is secured, the support portion of the intraocular lens can be easily engaged with the groove of the inhibiting device and easily deformed when the inhibiting device is inserted into the capsular bag. For instance, the inner diameter of the back wall, wherein the above circular hole is formed, is preferably 5 to 7.5 mm in accordance with the object of the present invention.

In the present invention, the inhibiting device is not limited to the device wherein the shape in section is square.

It will be explained with reference to Fig. 10 as follows how to use the inhibiting device 11. Firstly, the inhibiting device 11 is inserted into the capsular bag. The inserting method is the same as mentioned hereinbefore. Secondly, an intraocular lens 6 is inserted into the capsular bag. At this time, the support member of the intraocular lens is contacted with the slanting surface of the back wall 13. Then the support member moves along the slanting surface due to a restoring force thereof and extends to the groove 12 so that the support member is completely contained in the groove 12. In other word, the back wall 13 which is centripetally protruded plays such a part, as to guide the support member of the intraocular lens 6 so that the intraocular lens can be easily and surely inserted and fixed. Since the back wall 13 is centripetally protruded, an ophthalmologist who performs an operation can not only confirm the back wall 13 of the inhibiting device which is inserted into an eye and set but also insert and fix the intraocular lens easily and surely.

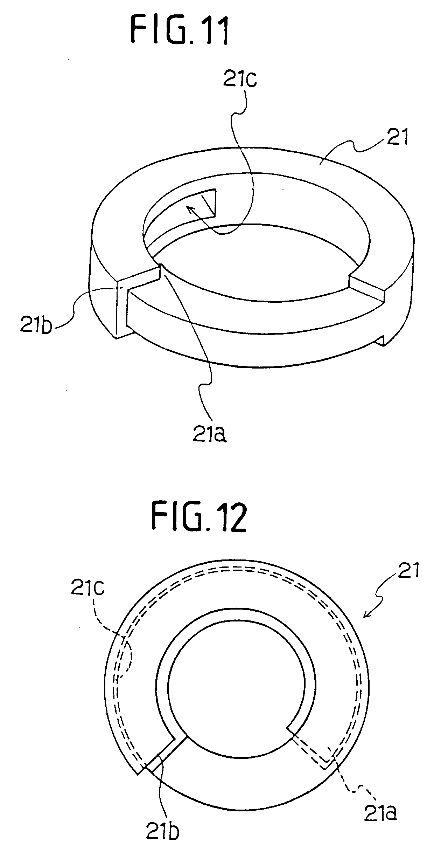

In Fig. 11, another embodiment of the inhibiting device which does not have the groove, is shown.

The inhibiting device 21 is an example of a variation of the inhibiting device 1 shown in Fig. 1. The inhibiting device has such a construction that one end 21a of the inhibiting device is inserted into a groove 21c which is formed from the other end 21b.

Thus constructed inhibiting device 21 can shorten the outer diameter thereof in such a manner that one end 21a is inserted into the groove 21c from the other end 21b. Therefore, the inhibiting device can be still more easily inserted into the eye. By inserting the inhibiting device into the capsular bag, the inhibiting device is completely inscribed in the equator of the capsular bag, the residual epithelial cells in the equator of the capsular bag can be sealed in the equator and cataract caused by proliferation of cells can be effectively inhibited. Further, since the inhibiting device can keep the shape of the capsular bag circular, shrinkage of the capsular bag is inhibited and aftercataract can be effectively inhibited.

On the other hand, since the device can keep the shape of the capsular bag circular, the shape of the incised opening is kept ink good state. Therefore, when the intraocular lens is inserted after the crystalline lens is removed, the intraocular lens can be easily inserted and it does not happen that the intraocular lens damages the incised opening by adding unstable force. In this case, the intraocular lens can be firmly retained and inhibited from falling down and moving, then can be fixed in good state by using an inhibiting device having a groove.

## Claims

1. Device for inhibiting aftercataract, the device (1; 2; 4; 11) being made of resilient material and having a substantially ring-like shape, characterized in that the device comprises two corner portions (1a; 2a; 3a) protruding along the whole outer periphery of the device for internally touching the equator of a capsular bag along the whole outer periphery thereof.

2. Device of claim 1, characterized in that the outer periphery has a recess which is grooved centripetally between the corner portions (2a).

3. Device of claim 1 or 2, characterized in that it is provided with a groove (5; 12) for engaging with a support member of an intraocular lens (6) extending in the circumferential direction at the inner periphery of the device in order to retain the intraocular lens (6).

4. Device of claim 3, characterized in that the groove (5; 12) is formed in the whole inner periphery of the device (4; 11).

5. Device of claim 4, characterized in that the groove (12) comprises a front wall portion (14) and a back wall portion (13) forming protrusions circumferentially at the inner periphery of the device (11) in such a manner that the back wall portion (13) is extending more toward the center of the device than the front wall portion (14).

6. Device of claim 5, characterized in that the back wall portion (13) is bevelled in such a manner that it tapers toward a central axis of the device (11).

## Patentansprüche

1. Vorrichtung zum Verhindern von Sekundärkatarakt, wobei die Vorrichtung (1; 2; 4; 11) aus elastischem Material hergestellt ist und eine im wesentlichen ringähnliche Form aufweist, **dadurch gekennzeichnet,** daß die Vorrichtung zwei entlang des gesamten äußeren Umfangs der Vorrichtung vorspringende Eckbereiche (1a; 2a; 3a) für eine entlang deren gesamten äußeren Umfangs innere Berührung des Teilungskreises einer Kapsel-Tasche aufweist.

2. Vorrichtung nach Anspruch 1, dadurch gekennzeichnet, daß der äußere Umfang eine Ausnehmung aufweist, die zentripetal zwischen den Eckbereichen (2a) genutet ist.

3. Vorrichtung nach Anspruch 1 oder 2, dadurch gekennzeichnet, daß sie mit einer für den Eingriff mit einem Tragelement einer intraokularen Linse (6) sich in Umfangsrichtung an dem inneren Umfang der Vorrichtung erstreckenden Nut (5, 12)versehen ist, um die intraokulare Linse (6) zurückzuhalten.

4. Vorrichtung nach Anspruch 3, dadurch gekennzeichnet, daß die Nut (5; 12) in dem gesamten inneren Umfang der Vorrichtung (4; 11) ausgebildet ist.

5. Vorrichtung nach Anspruch 4, dadurch gekennzeichnet, daß die Nut(12) einen vorderen Wandungsbereich (14) und einen hinteren Wandungsbereich (13) aufweist, die pheripherisch Vorsprünge an dem inneren Umfang der Vorrichtung (11) auf eine Weise bilden, so daß der hintere Wandungsbereich (13) sich mehr in Richtung auf die Mitte der Vorrichtung erstreckt als der vordere Wandungsbereich (14).

6. Vorrichtung nach Anspruch 5, dadurch gekennzeichnet, daß der hintere Wandungsbereich (13) auf eine Weise abgeschrägt ist, so daß er sich in Richtung auf eine zentrale Achse der Vorrichtung (11) verjüngt.

## Revendications

1. Dispositif pour inhiber la cataracte secondaire, le dispositif (1 ; 2 ; 4 ; 11) étant fait d'une matière élastique et ayant une forme sensiblement annulaire, caractérisé en ce que le dispositif comprend deux parties d'angle (1a ; 2a ; 3a) qui font saillie le long de toute la périphérie extérieure du dispositif pour toucher intérieurement l'équateur d'un sac capsulaire le long de toute sa périphérie extérieure.

2. Dispositif selon la revendication 1, caractérisé en ce que la périphérie extérieure présente un évidement qui est creusé dans la direction centripète entre les parties d'angle (2a).

3. Dispositif selon la revendication 1 ou 2, caractérisé en ce qu'il est muni d'une gorge (5 ; 12) destinée à coopérer avec un élément de support d'une lentille intra-oculaire (6) et qui s'étend dans la direction circonférentielle à la périphérie intérieure du dispositif afin de retenir la lentille intra-oculaire (6).

4. Dispositif selon la revendication 3, caractérisé en ce que la gorge (5 ; 12) est formée dans toute la périphérie intérieure du dispositif (4 ; 11).

5. Dispositif selon la revendication 4, caractérisé en ce que la gorge (12) comprend une partie de paroi avant (14) et une partie de paroi arrière (13) qui forment des saillies circonférentiellement sur la périphérie intérieure du dispositif (11) de telle manière que la partie de paroi arrière (13) s'étende plus loin en direction du centre du dispositif que la partie de paroi avant (14).

6. Dispositif selon la revendication 5, caractérisé en ce que la partie de paroi arrière (13) est biseautée de manière à présenter une section décroissante vers l'axe central du dispositif (11).
